# EUROPEAN PATENT SPECIFICATION

(11) **EP 2 548 886 B1**
(45) Date of publication and mention of the grant of the patent: **30.08.2017**
(21) Application number: 11175002.2
(22) Date of filing: 22.07.2011
(51) Int. Cl.: C07K 14/435, C12Q 1/68

(54) **Genetic test for genetic disposition of haemophilia A in Havanese dogs**
Gentest für die genetische Disposition von Hämophilie A bei Havanesern
Test génétique pour disposition génétique de l'hémophilie A chez les chiens havanais

(43) Date of publication of application: 23.01.2013
(73) Proprietor: Laboklin GmbH & Co. KG, 97688 Bad Kissingen (DE)
(72) Inventor: Müller, Elisabeth Dr., 97688 Bad Kissingen (DE); Kühnlein, Petra Dr., 97456 Dittelbrunn (DE); Kehl, Alexandra, 97702 Wermerichshausen (DE)
(74) Representative: advotec.

(56) References cited:
- CA-A1- 2 264 431
- HOUGH CHRISTINE ET AL: "Aberrant splicing and premature termination of transcription of the FVIII gene as a cause of severe canine hemophilia A: Similarities with the intron 22 inversion mutation in human hemophilia", THROMBOSIS AND HAEMOSTASIS, vol. 87, no. 4, April 2002 (2002-04), pages 659-665, XP9154546, ISSN: 0340-6245
- BROOKS M B ET AL: "Indirect carrier detection of canine haemophilia A using factor VIII microsatellite markers", ANIMAL GENETICS, vol. 39, no. 3, June 2008 (2008-06), pages 278-283, XP002665018, ISSN: 0268-9146
- MINNICK M F ET AL: "A highly repetitive DNA sequence possibly unique to canids", GENE, ELSEVIER, AMSTERDAM, NL, vol. 110, no. 2, 15 January 1992 (1992-01-15), pages 235-238, XP023542493, ISSN: 0378-1119, DOI: 10.1016/0378-1119(92)90654-8 [retrieved on 1992-01-15]

## Description

The invention relates to a genetic test for genetic disposition of haemophilia A in Havanese dogs by genotyping the canine factor VIII gene.

### Introduction

Haemophilia A is a genetic bleeding disorder caused by a coagulation factor VIII (FVIII) deficiency and is characterised by spontaneous haemorrhage (particularly into joints) and excessive bleeding after trauma or surgery. Canine haemophilia A represents a naturally occurring hereditary coagulopathy with a range in severity of clinical signs and coagulant activity in different breed variants. It has been reported in many breeds (Antonarakis, 1995).

The human gene is located on the X chromosome (Xq28) and the gene spans 186 kb across 26 exons, coding for a ∼9kb mRNA. More than 40 % of all cases of severe haemophilia A in humans are caused by gross gene rearrangements of DNA sequence involving the FVIII gene. In addition, sequence deletions, insertions and several hundred single base substitutions/point mutations have been described of which approximately 100 lead to creation of preliminary peptide chain termination or STOP codons (Oldenburg and El-Maarri, 2006; http://europium.csc.mrc.ac.uk/WebPages/Main/main.htm; http://hadb.org.uk/WebPages/Main/main.htm).

The canine FVIII gene is a highly conserved gene coding for a ∼7kb mRNA (Cameron et al., 1998). Until now very few causative mutations have been described in the canine FVIII gene, among others is an aberrant spliced mRNA that terminates with a novel sequence element, showing a striking similarity with the intron 22 inversion found in approximately 45% of severely affected human haemophilia A patients (Hough et al., 2002). A further mutation was found in the Chapel Hill colony of FVIII-deficient dogs. The FVIII transcript shows an abnormal sequence following exons 1-22 in place of exons 23-26 that leads to the first stop codon after 22 amino acids after the canine FVIII Met 2116 (Lozier et al., 2002). In recent experiments a point mutation in the FVIII gene in a dog suffering from severe FVIII deficiency was detected (Mischke et al., unpublished). The aim of the present study was to define the genetic background of a mild haemophilia A in the Havanese.

Without known genetic background, especially diagnosis of female carriers of the defect allele is difficult. Apart from analyses on littermates it is mainly based on factor measurements. It is described that under less optimal conditions (e.g., sample shipment to the laboratory, less optimally calibrated test), carrier detection based on factor VIII activity measurements is often unreliable. The developed assay specific for the detected mutation is therefore a valuable tool for reliable detection of carriers, which is essential for effective breeding hygiene programs.

It is the object of the present invention to provide a genetic test for a method for analysing of a genotype of a Havanese dog assigned to haemophilia A.

This aim is achieved by the inventions as claimed in the independent claims. Advantageous embodiments are described in the dependent claims.

The problem is solved by providing a canine factor VIII gene of a Havanese dog, which comprises a SINE insert. SINEs (short interspersed element) are short DNA sequences between 50 and 500 base pairs, which are inserted into genes on various locations. These sequences are typically derived from t-RNA.

It has been found that an insert in the canine factor VIII gene leads to a strong reduction in the activity of the factor and could be assigned to haemophilia A in the dog. Often this insert leads to a truncation of the protein, due to stop codons present in the insert.

The canine factor VIII gene from Havanese dogs is a gene, for which the unmodified sequence is shown in Seq.-ID No. 1. The (unmodified) canine factor VIII gene according to invention is a sequence showing at least 97 %, 98 %, 99 % or 99.99 % sequence homology with the Seq.-ID No. 1. In a preferred embodiment the unmodified canine factor VIII gene has the sequence of Seq.-ID No. 1.

"Sequence homology" is a property of nucleotide sequences having a percentage of nucleotides identical at corresponding positions which is higher than in purely random alignments. Two sequences are considered as homologous when they show between them a minimum of homology (or sequence identity) defined as the percentage of identical nucleotides found at each position compared to the total nucleotides, after the sequences have been optimally aligned taking into account additions or deletions (like gaps) in one of the two sequences to be compared.

In a preferred embodiment of the invention the insert comprises a sequence having at least 97 %, 98 %, 99 % or 99.99 % sequence homology to one of the Seq.-ID Nos. 2 to 4. In a more preferred embodiment of the invention the insert comprises a sequence according to one of the Seq.-ID Nos. 2 to 4.

In another preferred embodiment of the invention the insert is located in exon 14 of canine factor VIII gene. It is located starting from position 2095 to 5562 of the canine factor VIII gene according to Seq.-ID No. 1.

In a more preferred embodiment of the invention the insert is located after position 580 of exon 14 of canine factor VIII gene or position 2675 of Seq.-ID No. 1.

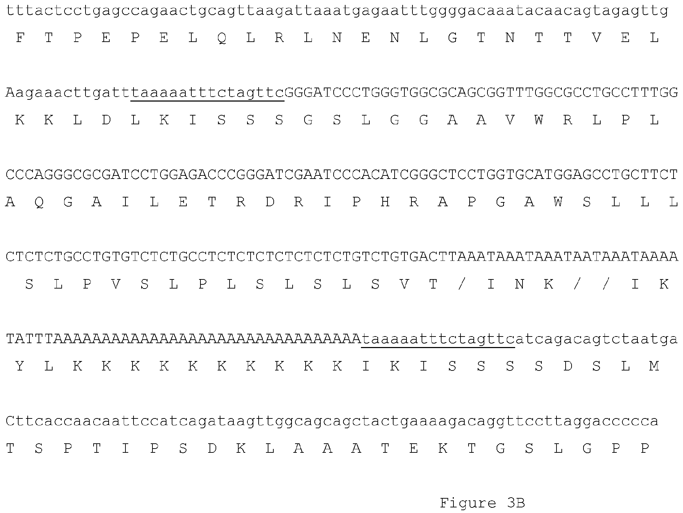

The canine factor VIII gene comprises a sequence having at least 97 %, 98 %, 99 % or 99.99 % sequence homology to Seq.-ID No. 5. In a more preferred embodiment of the invention the canine factor VIII gene comprises a sequence according to Seq.-ID No. 5. This sequence shows a part of the sequence of exon 14, wherein the SINE insert is located within the exon. This sequence is the same sequence as shown in figure 3B.

The invention therefore relates to a method for analysing by a genetic test to genotyping a Havanese dog assigned to haemophilia A by identification of at least one insert in the canine factor VIII gene of the genome of a Havanese dog, wherein the insert is a SINE insert.

In a preferred embodiment the insert comprises a sequence having at least 97 %, 98 %, 99 % or 99.99 % sequence homology to one of the Seq-ID Nos. 2 to 4. In a more preferred embodiment of the invention the insert comprises a sequence according to one of the Seq.-ID Nos. 2 to 4.

In a preferred embodiment the genetic test identifies the insert within exon 14 of canine factor VIII gene, preferably at position 580 of exon 14 or position 2675 of Seq.-ID No. 1.

The canine factor VIII is analyzed for a sequence having at least 97 %, 98 %, 99 % or 99.99 % sequence homology to Seq.-ID No. 5. In a more preferred embodiment of the invention the canine factor VIII is analyzed for a sequence according to Seq.-ID No. 5.

The unmodified canine factor VIII gene used in this test has preferably a sequence showing at least 97 %, 98 %, 99 % or 99.99 % sequence homology with the Seq.-ID No. 1. In a preferred embodiment the unmodified canine factor VIII gene has the sequence of Seq.-ID No. 1.

The procedures to detect an insert in a genome are known to the person skilled in the art. Possible methods are sequencing the region of the genome, where the mutation is localised, with specific primers and then comparing the sequence with the wild type sequence or comparing the size of the obtained PCR product. The sequencing may be done directly with the genomic DNA, mRNA or cDNA further including amplification of the selected region and then sequencing the amplified sequence.

Other possible methods comprise ligase chain reaction (LCR), restriction fragment length polymorphism (RFLP), mass spectroscopy (MALDI-TOF), SSCP, DGGE, pyrosequencing, dHPLC, allele-specific hybridization, molecular beacons, microarrays, Flap endonuclease (FEN), real-time PCR (RT-PCR), quantitative PCR (qPCR), allele specific PCR or Taqman assay.

For example PCR may be used for the amplification of the region of the insert, preferably the region of Seq-ID No. 1, which contains at least one insert as described before.

The PCR can be based on the DNA, genomic DNA, mRNA or cDNA of the canine factor VIII gene. This may require further steps, for example transcribing the mRNA from the sample to cDNA and then quantifying the amount of cDNA comprising the insert in relation to other housekeeping genes and/or unmodified canine factor VIII gene.

It can be sufficient to choose primer pairs, which consist out of one primer binding to the regular canine factor VIII gene and one primer binding to the insert.

By this analysis it is possible to identify not affected, affected dogs as well as carrier dogs. These are dogs, which are heterozygous with respect of the mutation.

Another embodiment of the invention is an in vitro method for the determination of the genetic disposition for haemophilia A of a Havanese dog by the use of a canine factor VIII gene with at least one insert as described previously.

In another embodiment of the invention this process may be used for the selection of a Havanese dog for breeding.

In another aspect the invention relates to a kit for detection the preposition of a Havanese dog to haemophilia A comprising means for detecting a SINE insert in the canine factor VIII gene.

The kit comprises means for performing a PCR reaction for the detection of the insert in the canine factor VIII, preferably primers, polymerases and fluorescent dyes or probes, e.q. for Taqman assay. It may further comprise multiple sets for PCR reaction for a relative quantification. The total amount of cDNA may for example be determined from measuring canine housekeeping genes like beta-actin and/or GAPDH (Glyceraldehyde-3-phosphate dehydrogenase).

The kit comprises a pair of primers for the insert, from which at least one primer binds the insert, a pair of primers selective for FVIII and at least one pair of primers for one housekeeping gene.

Examples for corresponding primer pairs are given in the Seq.-ID Nos. 6 to 13. Seq.-ID Nos. 6 and 7 are FVIII specific primers. Seq.-ID Nos. 8 and 9 are primers for the 5'-exonic sequence and the SINE insert. Seq.-ID Nos. 10 and 11 are for beta-actin and Seq.-ID Nos. 12 and 13 are for GADPH.

In a preferred embodiment the SINE insert to be detected is a SINE insert as described for the canine factor VIII gene previously with respect to its sequence and location in the canine factor VIII gene.

Even if no multiple back-referenced claims are drawn, all reasonable combinations of the features in the claims shall be disclosed.
- Table 1: Sequence analysis of the canine FVIII DNA for the insert in exon 14 in Havanese dogs with different status of haemophilia A (N = normal, C = carrier, A = affected)
- Figure 1: Structure of the canine Factor VIII gene. 26 exons are indicated by vertical bars;
- Figure 2: PCR amplification of genomic DNA from a haemophilic (lane 1), a carrier (lane 2) and a healthy dog (lane 3). The size marker in line 4 is a 100bp ladder;
- Figure 3A: Location of the SINE insertion in exon 14.
- Figure 3B: Sequence of the SINE insert. The nucleotide sequence of the SINE is depicted in large black letters. Small black letters correspond to the factor VIII exon 14 sequence. The 16bp direct duplication sites at the insertion site and at the end of the insert is underlined. The predicted amino acid sequence is shown in below the nucleic acid sequence data. Stop codons are depicted as slash.
- Figure 3C: Comparison of the found SINE insertion (lower line) with CanB (upper line; Vassetzky 2002).
- Figure 4: Expression level of FVIII mRNA in healthy and affected dogs measured by quantitative RT-PCR. The mRNA expression levels of FVIII (normal or with SINE-insert in affected dogs) were estimated in relation to two canine house-keeping genes (ß-actin and GAPDH). The white bar depicts the mRNA for the FVIII gene without insert. The black bar shows results for the same mRNA with SINE- insert;
- Figure 5: Template availability for Factor VIII as given by Swissmodell: the lower bars highlight the available templates for homology modelling. These areas are also the only two parts in the protein sequence with predicted protease activity, as further analysis of the sequence of the middle area with SwissModell and LOMETS showed.
- Figure 6: Active site predictions for the mutated Factor VIII. Shown here are two different views (A and B) on the N-terminus of FVIII protein as modelled by SwissModell, as only this part is still expressed when the SINE- insert is present. The accessible surface areas of the predicted protease activity binding pockets for substrates (by 3D2Go) are shown with dark grey and light grey colouring. The circles indicate the active site of the binding pocket in both views. The main chain and the secondary structure is given in a ribbon display

### DETAILED DESCRIPTION OF THE INVENTION

### Materials and methods

### 2.1 Study design

The study included 2 phenotypically affected male dogs, 3 suspected carriers, and 29 healthy Havanese dogs. From all dogs with known or suspected haemophilia A status, factor VIII activity was measured to verify the status of haemophilia A. DNA from all these dogs was isolated from EDTA blood, and the coding region for canine factor VIII was amplified and sequenced. The obtained DNA sequence was compared with the wild type canine factor VIII DNA (Gene Bank Accession Number: AF016234). In order to prove the functional relevance of the detected insert, the expression of the defect allele was investigated by mRNA studies and the sequence of the insert was analysed for its consequences on translation.

To investigate, if the mutation is just a common SINE insert unrelated to haemophilia A, screening for the SINE mutation was performed in DNA samples of 18 Havanese with undefined haemophilia A status and samples of four other dog breeds (Doberman Pinscher: n=20; German Wire haired Pointer: n=25; Labrador: n=25, Malteser: n=5).

### 2.2 Animals and sample material

Havanese dogs with known status of haemophilia A were presented as patients or for haemophilia diagnosis to the Small Animal Clinic, University of Veterinary Medicine Hannover. The two phenotypically affected male Havanese dogs had all a known mild bleeding history and moderately reduced factor VIII activities of approx. 10 % (table 1). Clinical signs mainly included severe bleeding after minor surgeries and injuries and occasionally spontaneous bleeding. Among the two bitches with suspected carrier status due to moderately reduced factor VIII activity (48-69 %), two were confirmed by affected littermates. 29 healthy Havanese dogs showed factor VIII activity within the reference range and no hint for an excessive bleeding within their history.

### 2.3 Blood sample collection

Citrated blood for factor VIII activity measurement was obtained from a cephalic or saphenous vein using sterile disposable needles (1.1 x 30 mm) and only slight pressure was used to raise the vein. Citrated blood was collected into plastic tubes containing one part (1 mL) 0.11 mol/l sodium citrate solution and nine parts (9 mL) of blood, and immediately mixed by careful rocking. Platelet poor citrated plasma for factor VIII measurements was gained by centrifugation twice for 10 min at 2000 x g. The final supernatant was then collected and stored in aliquots at -70 °C. In addition, 5 mL of EDTA blood was collected for genetic testing.

### 2.4 Factor VIII activity measurements

Coagulation factor VIII activities were automatically measured using coagulometric tests based on commercial human deficient plasma in an AMAX Destiny coagulation analyser (ball coagulometer method; Trinity Biotech). A mixture of 20 µl of diluted citrated plasma (1:40 diluted with imidazole buffer, Siemens Healthcare Diagnostics), 20 µl of factor VIII deficient plasma (Siemens Healthcare Diagnostics), 20 µl activating reagent (PTT reagent, Diagnostica Stago) was incubated, and exactly after 3 minutes 20 µl 25 mmol/L CaCl₂ solution (Diagnostica Stago) was added as a starting reagent. A calibration curve was prepared with different dilutions of a canine pooled plasma corresponding to 200, 150, 125, 100, 75, 50, 25, 10, 5, and 1 % (% by volume). Canine pooled plasma was prepared by mixing identically citrated plasma aliquots from 100 clinically healthy dogs with unremarkably haematological and biochemical profile and its factor VIII activity was defined as 100%. All results are mean values of measurements in duplicate. The reference range for this test is 75-140 %.

### 2.5 DNA isolation

Genomic DNA was extracted from 200 µl EDTA whole blood using a commercially available kit Biosprint 15 DNA Blood Kit (Qiagen) following manufacturer's instructions.

### 2.6 mRNA Isolation and cDNA synthesis

Whole RNA was extracted from 200 µl EDTA whole blood using QIAamp RNA Blood Mini Kit (Qiagen) including DNase digestion with RNase-free DNase kit (Qiagen) following manufacturer's instructions. Thereafter, the RNA samples were transcribed into cDNA using the High Capacity cDNA Reverse Transcription kit (Applied Biosystems).

### 2.7 DNA amplification

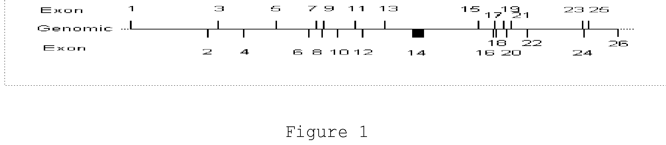

Canine factor VIII cDNA sequence was utilised to search the dog genome database at NCBI (http://www.ncbi.nlm.nih.gov/genome/guide/dog) and the 26 exons of the canine factor VIII gene were determined. 40 pairs of oligonucleotide primers (sequences and PCR conditions available on request) were used to amplify the coding region and the exon-intron boundaries of the canine factor VIII gene using genomic DNA of the dogs. Target sequences were amplified in 50 µl reaction mixtures under standard reaction conditions containing approximately 50 ng genomic DNA and 0.2 µmol L⁻¹ of each primer. Figure 1 shows the different exons of the gene.

### 2.8 DNA sequencing

PCR products were purified prior to sequencing using Min Elute PCR Purification Kit (Qiagen). Sequencing was done by cycle sequencing using DyeDeoxy Terminators (Applied Biosystems) in an automated sequencer ABI 3130 Genetic Analyzer (Applied Biosystems). Sequence comparison was performed with different groups of Havanese with defined haemophila A status using BLAD ClustalW alignment (http://www.ebi.ac.uk/Tools/clustalw2/index.html) and BLAST (http://blast.ncbi.nlm.nih.gov/Blast.cgi).

### 2.9 mRNA pattern analysis of the exon 14

To determine the effect of the insert on the transcription of exon 14 the mRNA pattern of exon 14 of haemophilic dogs, suspected carrier dogs as well as healthy dogs was analysed. The target sequence was amplified in 50 µl reaction mixtures under standard reaction conditions containing cDNA and 0.2 µmol L⁻¹ of each primer flanking the insert region. PCR products were analysed with gel electrophoresis and sequenced.

### 2.10 Analysis of the insert

The sequence of the insert was screened for stop codons and frame shift. Furthermore, homologies to known insert such as Alu were investigated.

### 2.11 Quantitative analysis of mRNA expression levels

Quantitative real-time RT-PCR (qPCR) was used to estimate the relative gene expression levels of FVIII in different animals (Klein 2002). FVIII specific primers were designed for exon 14 of the gene (fwd: CAACATGATGGTATGGAAGCTTATG (Seq.- ID No. 6), rev: CAAAGCTAACTACGTCCATGTCAGA (Seq.- ID No. 7)). Additionally, a second qPCR assay was developed for the quantification of insert-containing mRNA which employs a reverse primer targeting the SINE insertion as well as a forward primer binding to the 5' exonic sequence (fwd: GAGAATTTACTCCTGAGCCAGAACT (Seq.- ID No. 8), rev: CCAGGGATCCCGAACTAGAAAT (Seq.- ID No. 9)). The reaction efficiency of both assays were determined as previously described (Klein et al. 1999). QPCR analysis was performed on a 7500 Real-Time PCR System utilizing the Power SYBR Green Master Mix (both Applied Biosystems). The 20 µl qPCR reaction mixes included 300 nM of each primer and 2 µl cDNA template. The PCR profile started with initial denaturation at 95°C for 10 min succeeded by 40 cycles consisting of 95°C for 15 sec and 60°C for 1 min, followed by a continuous increase of temperature from 60°C to 95°C for melting curve analysis. Total cDNA amounts in the samples were quantified by qPCR amplification of two canine housekeeping-genes (Jais et al. 2011): beta-actin (fwd: TCGCTGACAGGATGCAGAAG (Seq.- ID No. 10), rev: GTGGACAGTGAGGCCAGGAT (Seq.- ID No. 11)) and GAPDH (fwd: TCCACCCACGGCAAATTC (Seq.- ID No. 12), rev: GCATCACCCCATTTGATGTTG (Seq.- ID No. 13)) using the same reaction conditions. The obtained Ct-values for FVIII and insert-containing FVIII mRNA were subsequently normalized to the mean values of both reference genes and relative expression numbers were calculated according to the 2^{-ΔΔC}_{T} analysis approach (Livak and Schmittgen, 2001).

### 2.12 Protein Modeling

Analysis of the DNA sequence with the incorporated SINE insert showed the existence of a stop codon at position 2835 (174 nucleic acids upstream after the beginning of the SINE-Insert). To obtain 3d protein models for further analysis of the effects of this stop codon, homology modelling through SwissModell (Arnold, 2006; Kiefer, 2009; Schwede 2003, Guex, 1997 and Peitsch, 1995) was applied. SwissModell provides high quality modelling and also provides protein sequence alignments as well as residual RSMD. The calculated model showed only a 3.7Å deviation to the template structures (2r7eA and 2r7eB).

### 3 Results

### 3.1 Identification of the mutation

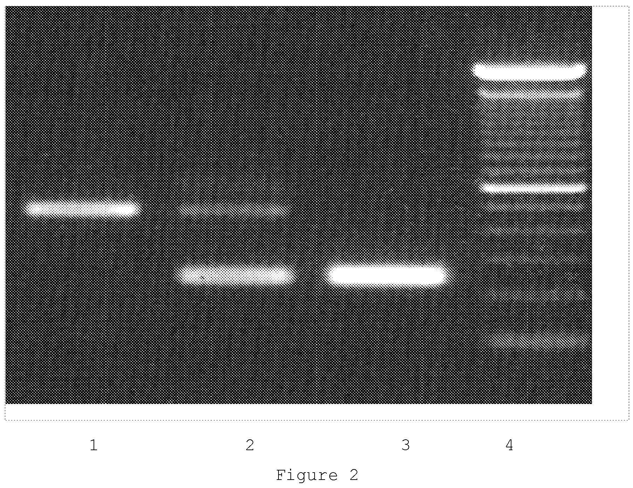

After amplification of a part of exon 14 we observed differences in length of the PCR products: when using DNA of a healthy dog we yielded a product with the expected length of 250bp whereas using DNA of a clinically affected dog resulted in a PCR product with the length of 450bp. Both products could be amplified out of DNA from suspected carriers (Fig. 2).

These amplicons segregated with the haemophilic phenotype among the aforementioned dogs: All affected dogs were homozygous for the detected mutation in exon 14. Two dogs, which were suspected or proven carriers, were found to be heterozygous for the insert mutation and none of the healthy Havanese was affected by the mutation. No further alterations in the sequences between affected dogs and the healthy control group could be observed.

Screening for this insert by PCR and gel electrophoresis did not reveal the presence of the insert in exon 14 of canine factor VIII DNA in 25 further Havanese dogs with undefined haemophilia A status and 75 animals of four other dog breeds.

### 3.2 Characterisation of the insertion

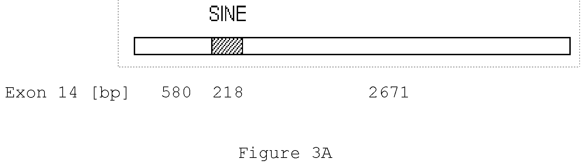

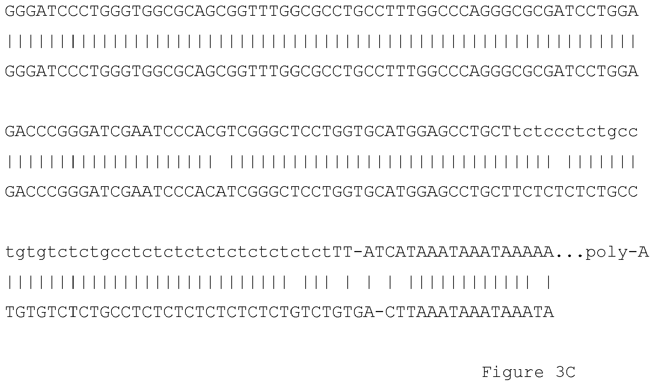

Sequence analysis of exon 14 showed the exact length of the insert being 218bp (Fig. 3A). The mutation occurs after nucleotide 2675 of the factor VIII cDNA and at nucleotide 580 of exon 14, respectively. The insert is flanked by a 16-bp target site duplication (Fig. 3B). The ORF of the insert contains 58 amino acids after canine factor VIII serine before the first of three stop codons (Fig. 3B). The insert was characterised as a tRNA-derived SINE, highly similar to the unique canine SINEs described by Minnick et al. 1991 and to CanB described by Vassetzky et al 2002 (Fig. 3C).

### 3.3 Analysis of the transcription pattern of exon 14

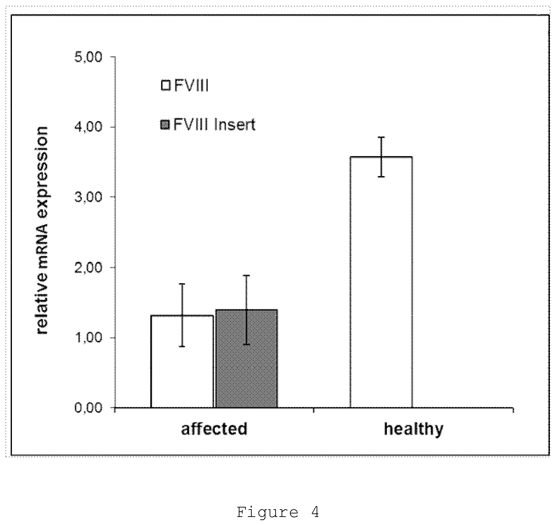

To investigate the effects of the found SINE insert on transcription and translation of the factor VIII gene, exon 14 was amplified by qPCR using the region in front of the SINE and the SINE itself as primer targets, respectively and RNA from EDTA blood samples of a healthy and an affected dog as templates. Relative quantification was applied to determine the expression level of FVIII mRNA comparing healthy and affected dogs. In the samples from the healthy dogs only the normal mRNA is detectable. No SINE cDNA can be found in these samples. In the samples from the affected dogs less exon 14 mRNA is produced and the whole amount of exon 14 mRNA contains the insert (Figure 4).

### Protein Modeling

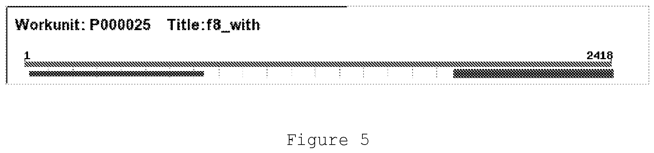

As was shown by our modelling approaches, the DNA could be translated into a fully functional protein if the SINE- Insert is not present. When present only a part of the protein was translated. An overview of the areas coding for the functional structures of the protein is given in figure 5. One can also see that the middle part - in which the SINE- Insert is located - does not seem to have a particular function directly associated with Factor VIII subunit A or B. Further ab initio modelling with LOMETS (a local meta-threading server, Zhang, 2007) showed that this part forms a circular structure and thus could function as a linker between the two functional parts.

Analysis of subunit A of Factor VIII with 3D2Go (part of the Phyre suite, Kelley, 2009) showed that subunit A (upstream to the insert) shows two areas with propable active sites.

Thus we predict that even with the SINE insert present not all functionality of the protein is lost and some residual activity should be measured even in affected individuals.

### 4. Discussion

Comparison of the entire coding region of the canine factor VIII DNA sequence and the exon-intron junctions of two haemophilic dogs with severe factor VIII deficiency with the wild type canine factor VIII DNA revealed an insertion at nucleotide 2675 in exon 14 of the haemophilic factor VIII gene.

The presence of the mutation was confirmed in 5 dogs (2 affected, 3 carriers) with defined haemophilia A status. In addition, a cohort of samples from different breeds did not show the observed mutation. The latter results strongly suggest that the detected mutation cannot be a common and unimportant mutation.

SINEs are described as a potential source of diseases in humans as well as in dogs. In humans, Alu insertions associated with a disease are usually found in *coding* sequences or near the exon-intron boundaries affecting splicing patterns (Druker 2004). Similarily, SINE insertions have been reported as a causative mutation in dogs. A SINE within exon 2 of the PTPLA gene generates aberrant splicing patterns associated with Hereditary myopathy in Labrador Retrievers (Pele 2005). Narcolepsy in Dobermans segregates with a SINE insertion in the 5'-flanking intronic region of exon 4 of HCRTR2. This insertion displaces a putative lariat branch point sequence necessary for proper splicing (Lin 1999). In merle dogs, a SINE insertion was found at the intron10-exon11 boundary in the SILV gene (Clark 2006).

The SINE found in haemophilic Havanese dogs lies within the coding sequence of the factor VIII gene. As shown in Fig.3 the FVIII gene is transcribed together with the SINE in affected dogs. The amino acid sequence will be altered due to the different nucleotide sequence, additionally the SINE contains several stop codons (Fig. 3B).

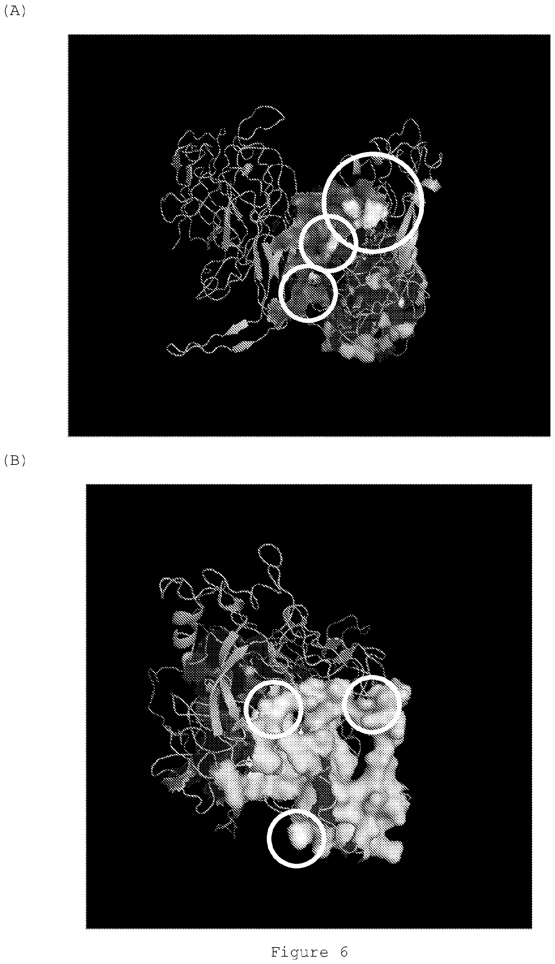

During translation, this unnormal mRNA will most probably lead to a truncated protein, however some residual activity is predicted to be present due to the N-terminus of FVIII being intact (Fig. 6).

Insertions in the factor VIII gene are also described as cause of Haemophilia A in humans. In the official database two LINEs in exon 14 and one Alu repeat insertion in exon 14 are listed (http://hadb.org.uk/). Additionally, an insertion with a length of 280bp in exon 14 is described in the database of the Institut für Humangenetik Uni Würzburg (personal communication with Dr. Hasenmüller).

The results of the present study reveal the detected SINE insertion in exon 14 as a responsible mutation for Haemophilia A in Havanese dogs with sufficient certainty.

Unless the fact, that none of the 25 unselected Havanese dogs were tested positive for the mutation, investigation of larger numbers of dogs of this breed seems valuable to define the actual prevalence of the disease.

The described embodiments are considered in all respects to be illustrative and not restrictive. The scope of the inventions are, therefore, indicated by the appended claims, rather than by the foregoing description. All changes which come within the meaning and range of equivalence of the claims are to be embraced within their scope.

### References Cited

Antonarakis, S.E., 1995. Molecular genetics of coagulation factor VIII gene and hemophilia A. Thromb. Haemost. 74, 322-328.
Cameron C., Notley C., Hoyle S., McGlynn L., Hough C., Kamisue S., Giles A. And Lillicrap D.; 1998: The canine factor VIII cDNA and 5' flanking sequence; Thromb Haemost 79(2): 317-322
Clark L.A., Wahl J.M., Rees C.A. and Murphy K.E.; 2006: Retrotransposon insertion in SILV is responsible for merle patterning of the domestic dog; PNAS 103(5): 1376-1381
Druker R. And Whitelaw E.; 2004: Retrotransposon-derived elements in the mammalian genome: a potential source of disease; J Inherit Metab Dis 27: 319-330
Hough, C., Kamisue, S., Cameron, C., Notley, C., Tinlin, S., Giles, A., Lillicrap, D., 2002. Aberrant splicing and premature termination of transcription of the FVIII gene as a cause of severe canine hemophilia A: similarities with the intron 22 inversion mutation in human hemophilia. Thromb. Haemost. 87, 659-65.
Jais, A., Klein, D., Wolfesberger, B., Walter, I. (2011). Gene expression profile of vascular endothelial growth factor and ist receptors in various cell types of the canine lymph node (using laser capture microdissection)..Veterinary Immunology and Immunopathology 140 (3-4): 207 - 214
Klein, D., Janda, P., Steinborn, R., Müller, M., Salmons, B., Günzburg, W.H. (1999). Proviral load determination of different feline immunodeficiency virus isolates using real-time polymerase chain reaction: Influence of mismatches on quantification. Electrophoresis 20: 291-299
Klein, D. (2002). Quantification using real-time PCR technology: applications and limitations. Trends in Molecular Medicine 8 (6): 257-260
Lin L., Faraci L, Kadotani H., Rogers W., Lin X., Qiu X., de Jong P.J., Nishino S. And Mignot E.; 1999: The sleep disorder canine narcolepsy is caused by a mutation in the hypocretin (orexin) receptor 2 gene; Cell 98(3): 365-376
Livak K.J., And Schmittgen T.D.; Analysis of Relative Gene Expression Data Using Real-Time Quantitative PCR and the 2-ΔΔCT Method. Methods 25: 402-408.
Lozier J.N., Dutra A.,Pak E., Zhou N., Zheng Z., Nichols T.C., Bellinger D.A., Read M. and Morgan R.A.; 2002: The Chapel Hill hemophilia A dog colony exhibits a factor VIII gene inversion; PNAS 99(20): 12991-12996
Minnick M.F., Stillwell L.C., Heineman J.M. And Stiegler G.L.; 1991: A highly repetitive DNA sequence possibly unique to canids; Gene 110: 235-238
Naylor J.A., Buck D., Green P., Williamson H., Bentley D. And Gianelli F.; 1995: Investigation of the factor VIII intron 22 repeated region (int22h) and the associated inversion junctions; Hum Mol Genet 4(7): 1217-1224
Oldenburg, J., El-Maarri, O., 2006. New insight into the molecular basis of hemophilia A. Int. J. Hematol. 83, 96-102.
Pele M., Tiret L., Kessler J.L., Blot S.and Panthier J.J.; 2005: SINE exonic insertion in the PTPLA gene leads to multiple splicing defects and segregates with the autosomal recessive centronuclear myopathy in dogs; Hum Mol Genet 14(11): 1417-1427
Vassetzky N.S. And Kramerow D.A.; 2002: CAN - a pan-carnivore SINE family; Mamm Genome 13: 50-57
Bentolila S., Bach J., Kessler J., Bordelais I., Cruaud C., Weissenbach J. And Panthier J.; 1999: Analysis of major repetitive DNA sequences in the dog (Canis familiaris) genome; Mamm Genome 10: 699-705
Bicocchi, M.P., Pasino, M., Lanza, T., Bottini, F., Boeri, E., Mori, P.G., Molinari, A.C., Rosano, C., Aquila, M., 2003. Analysis of 18 novel mutations in the factor VIII gene. Br. J. Haematol. 122, 810-817.
Bowen D.J., 2002: Haemophilia A and Haemophilia B: molecular insights Review; J Clin Pathol 55: 127-144
Bowen D.J.; 2003: Unleashing the long-distance PCR for detection of the intron 22 inversion of the factor VIII gene in severe haemophilia A; Thromb Haemost 89:201-202
Dunning M.D., Averis G.F., Pattinson H., Targett M., Cade S. And Herrtage M.E.; 2009: Haemophilia A (factor VIII deficiency) in a litter of Weimaraners; J Sm Anim Prac 50: 357-359
Holbrook, J.A., Neu-Yilik, G., Hentze, M.W., Kulozik, A.E., 2004. Nonsense-mediated decay approaches the clinic. Nat. Genet. 36, 801-808.
Lakich D., Kazazian H.H., Stylianos E.A. And Gitschier J.; 1993: Inversions disrupting the factor VIII gene are a common cause if severe haemophilia A; Nature Gen 5: 236-241
Oldenburg J., Schröder J., Graw J., Ivaskevicius V., Brackmann H.H., Schramm W., Müller C.R., Seifried E. And Schwaab E.; 2003: Bedeutung der Mutationsdiagnostik bei Patienten mit Hämophilie A; Hamosatseologie 23:6-12
Tuddenham E.G.D., Schwaab R., Seehafer J. Et al; 1994: Haemophilia A: database of nucleotide substitutions, deletions, insertions and rearrangements of the factor VIII gene, second edition; Nuc Acids Res 22(22): 4851-4868

**Table 1**

| **No.** | **Dog** | **sex (M/F)** | **age (years, months)** | **Factor VIII-activity (%)** | **Sequence analysis or screening test*** |
|---|---|---|---|---|---|
| 1 | P 1887 | M, affected | 1, 6 | 10 | A |
| 2 | P 1947 | M, affected | 3, 11 | 15 | A |
| 3 | N 985 | F, hidden carrier | 3, 4 | 80 | C |
| 4 | A 7009 | F, carrier (proven) | unknown | unknown | C |
| 5 | N 984 | F | 1, 11 | 91 | N |
| 6 | N 986 | F | 1, 11 | 109 | N |
| 7 | N 997 | M | 0, 2 | 81 | N |
| 8 | N 998 | M | 0, 2 | 86 | N |
| 9 | N 999 | W | 0, 2 | 80 | N |
| 10 | P 1889 | W | 1, 11 | 104 | N |
| 11 | N 1023 | M | 0, 2 | 74 | N |
| 12 | N 1024 | M | 0, 2 | 85 | N |
| 13 | N 1025 | M | 0, 2 | 75 | N |
| 14 | N 1026 | M | 0, 2 | 86 | N |
| 15 | N 1027 | M | 0, 2 | 86 | N |
| 16 | N 1028 | W | 0, 2 | 74 | N |
| 17 | N 1029 | M | 1, 8 | 91 | N |
| 18 | N 1030 | W | 6, 4 | 95 | N |
| 19 | N 1032 | M | 0, 1 | 74 | N |
| 20 | N 1033 | M | 0, 1 | 73 | N |
| 21 | N 1034 | W | 0, 1 | 68 | N |
| 22 | N 1035 | W | 0, 1 | 67 | N |
| 23 | N 1036 | M | 0, 1 | 68 | N |
| 24 | N 1037 | M | 0, 1 | 86 | N |
| 25 | N 1038 | F | 0, 1 | 67 | N |
| 26 | N 1039 | F | 0, 11 | 82 | N |
| 27 | N 1040 | F | 4, 9 | 83 | N |
| 28 | 159838 | M | 1, 5 | 109 | N |
| 29 | N1041 | M | 0, 11 | 90 | N |
| 30 | N1042 | M | 1, 6 | 113 | N |
| 31 | N1043 | F | 2, 4 | 130 | N |

| | | | | | |
|---|---|---|---|---|---|
| *N = normal, C = carrier, A = affected | | | | | |

## Claims

1. Canine factor VIII gene of a Havanese dog as shown in Seq.-ID No. 1, wherein the canine factor VIII gene is assigned to haemophilia A and comprises a sequence having at least 97 % sequence homology to Seq.-ID No. 5.

2. A method for analysing of a genotype of a Havanese dog assigned to haemophilia A by a genetic test for identification of at least one insert in the canine factor VIII, as shown in Seq.-ID No. 1, of the genome of the dog, wherein the canine factor VIII gene is analyzed for a sequence having at least 97 % sequence homology to Seq.-ID No. 5.

3. The method for analysing according to claim 2, wherein PCR is used for amplification of a region of the DNA, mRNA or cDNA of the canine factor VIII gene, which contains the insert.

4. An in vitro method for the determination of the genetic disposition for haemophilia A of a Havanese dog, **characterized by** the use of the canine factor VIII gene of the dog according to claim 1 and detection of a sequence having at least 97 % sequence homology to Seq.-ID No. 5.

5. Kit for detection the preposition of a Havanese dog to haemophilia A comprising means for performing a PCR reaction for the detection of the insert having at least 97% sequence homology to Seq.-ID No. 5 in the canine factor VIII gene according to claim 1, wherein the kit comprises a pair of primers for the insert, from which at least one primer binds the insert, a pair of primers selective for canine factor VIII and at least one pair of primers for one housekeeping gene.

## Patentansprüche

1. Canines Faktor-VIII-Gen eines Havaneser-Hundes nach Seq.-ID No. 1, wobei das canine Faktor-VIII-Gen mit Hämophilie A assoziiert ist und eine Sequenz umfasst, welche eine Sequenzhomologie von mindestens 97 % zu Seq.-ID No. 5 aufweist.

2. Verfahren zur Analyse eines Genotyps eines mit Hämophilie A assoziierten Havaneser-Hundes durch einen genetischen Test zur Identifizierung mindestens eines Inserts in dem in Seq.-ID No. 1 gezeigten caninen Faktor VIII des Genoms des Hundes, wobei das canine Faktor-VIII-Gen auf eine Sequenz hin analysiert wird, welche eine Sequenzhomologie von mindestens 97 % zu Seq.-ID No. 5 aufweist.

3. Verfahren zur Analyse nach Anspruch 2, wobei eine Region der DNA, mRNA oder cDNA des caninen Faktor-VIII-Gens, welche das Insert enthält, mittels PCR amplifiziert wird.

4. In-vitro-Verfahren zur Bestimmung der genetischen Disposition für Hämophilie A bei einem Havaneser-Hund, **gekennzeichnet durch** das Verwenden des caninen Faktor-VIII-Gens des Hundes nach Anspruch 1 und das Erkennen einer Sequenz, welche eine Sequenzhomologie von mindestens 97 % zu Seq.-ID No. 5 aufweist.

5. Kit zur Erkennung der Disposition eines Havaneser-Hundes für Hämophilie A, umfassend Mittel zur Durchführung einer PCR zur Erkennung des Inserts, welches eine Sequenzhomologie von mindestens 97 % zu Seq.-ID No. 5 aufweist, in dem caninen Faktor-VIII-Gen nach Anspruch 1, wobei das Kit ein Paar von Primern für das Insert, von denen mindestens ein Primer im Bereich des Inserts bindet, ein Paar von für den caninen Faktor VIII selektiven Primern und mindestens ein Paar von Primern für ein Housekeeping-Gen umfasst.

## Revendications

1. Gène du facteur VIII canin d'un bichon havanais tel que représenté dans la Seq.-ID No 1, dans lequel le gène du facteur VIII canin est associé à l'hémophilie A et comprend une séquence présentant une homologie de séquence d'au moins 97 % avec la Seq.-ID No. 5.

2. Procédé d'analyse d'un génotype d'un bichon havanais associé à l'hémophilie A par un test génétique pour l'identification d'au moins un insert dans le facteur VIII canin, tel que représenté dans la Seq.-ID No. 1, dans le génome du chien, dans lequel le gène du facteur VIII canin est analysé pour y détecter une séquence présentant une homologie de séquence d'au moins 97 % avec la Seq.-ID No. 5.

3. Procédé d'analyse selon la revendication 2, dans lequel la PCR est utilisée pour l'amplification d'une région de l'ADN, de l'ARNm ou de l'ADNc du gène du facteur VIII canin qui contient l'insert.

4. Procédé in vitro pour la détermination de la disposition génétique à l'hémophilie A d'un bichon havanais, **caractérisé par** l'utilisation du gène du facteur VIII canin du chien selon la revendication 1 et la détection d'une séquence présentant une homologie de séquence d'au moins 97 % avec la Seq.-ID No. 5.

5. Kit pour la détection de la disposition d'un bichon havanais à l'hémophilie A comprenant des moyens pour effectuer une réaction PCR afin de détecter l'insert présentant une homologie de séquence d'au moins 97 % avec la Seq.-ID No. 5 dans le gène du facteur VIII canin selon la revendication 1, dans lequel le kit comprend une paire d'amorces dont au moins une amorce lie dans la région de l'insert, une paire d'amorces sélectives pour le facteur VIII canin et au moins une paire d'amorces pour un gène domestique (anglais : *housekeeping gene*).


### SEQUENCE LISTING

<110> LABOKLIN GmbH & Co. KG
<120> Genetic test for genetic disposition of haemophilia A in Havanese dogs
<160> 13
<170> BiSSAP 1.0
<210> 1
   <211> 7032
   <212> DNA
   <213> Canis lupus familiaris
<220>
   <221> source
   <222> 1..7032
   <223> /mol_type="DNA" /note="AF016234 GenBank" /organism="Canis lupus familiaris"
<400> 1
   atgcaagtag agctctacac ctgctgcttt ctgtgccttt tgcccttcag ccttagtgcc      60
   accagaaaat actacctcgg tgcagtggaa ctgtcctggg actatatgca aagtgacctg     120
   ctcagtgcgc tgcacgcgga cacaagcttt tcttccaggg tgccaggatc tttgccactc     180
   accacgtcag tcacgtacag aaagactgtg tttgtagagt ttacagatga ccttttcaac     240
   attgccaagc ccaggccacc gtggatgggc ctgctgggtc ctaccatcca ggctgaggtt     300
   tatgacacag tggtcattgt ccttaagaac atggcttctc atcctgtcag ccttcacgct     360
   gttggtgtat cctattggaa agcttctgaa ggtgctgagt atgaggatca gaccagccaa     420
   aaggagaagg aagatgataa tgtcattcct ggtgaaagcc atacctatgt ctggcaggtc     480
   ctgaaagaga atggcccaat ggcctctgat ccaccatgtc tcacctactc atatttttca     540
   cacgtggacc tggtgaaaga cctgaattca ggcctcattg gagccctgct ggtttgcaaa     600
   gaagggagtc tggccaaaga aaggacacag accttgcagg aatttgtcct actttttgct     660
   gtatttgatg aagggaaaag ttggcactca gaaacaaatg cgtctttgac acaggctgag     720
   gcccagcatg agctgcacac catcaatggc tatgtaaaca ggtctctgcc aggtcttact     780
   gtgtgtcaca agagatcagt ctattggcat gtgattggaa tgggcaccac ccccgaagtg     840
   cactcaattt ttctcgaagg tcacacattt cttgtgagga accaccgcca ggcctccttg     900
   gagatctcac caattacttt ccttactgct cagacattcc tgatggacct tggccagttt     960
   ctactgtttt gtcatatccc ttcccatcaa catgatggta tggaagctta tgtcaaagta    1020
   gatagctgcc cagaggaacc ccagctgcgc atgaaaaata atgaagataa agattatgat    1080
   gatggtcttt atgattctga catggacgta gttagctttg atgacgacag ctcttctccc    1140
   tttatccaaa tccgctcagt tgccaagaag catcctaaaa cttgggtcca ctatattgct    1200
   gctgaggagg aggactggga ctatgctccc tcaggcccca cccccaatga tagaagtcat    1260
   aaaaatctgt atttgaacaa tggtcctcag cggattggta agaagtacaa aaaagtccga     1320
   tttgtggcat acacagatga gacatttaag actcgtgaag ctattcagta tgaatcagga     1380
   atcctgggac ctttacttta tggagaagtt ggagacacac tgctgattat atttaagaat     1440
   caagccagcc ggccatataa catctaccct catgggatca attatgtcac tcctctgcac     1500
   acagggagat tgccaaaagg tgtgaaacat ttgaaagata tgccaattct gccgggagag     1560
   atattcaagt ataaatggac agtgaccgta gaagatggac caactaaatc agatcctcgg     1620
   tgcctgaccc gatattactc aagcttcatt aatctggaga gagatctagc ttcaggactc     1680
   attggccctc ttctcatctg ctacaaagaa tctgtagatc aaagaggaaa ccagatgatg     1740
   tcagacaaga gaaatgtcat cctgttttct gtatttgatg agaatcgaag ctggtacctc     1800
   acagagaata tgcagcgctt cctccccaat gcagatgtag tgcagcccca tgacccagag     1860
   ttccaactct ctaacatcat gcacagcatc aatggctatg tttttgacaa cttgcagctg     1920
   tcagtttgtt tgcatgaggt ggcgtactgg tacattctaa gtgttggagc acaaactgac     1980
   ttcctgtctg tcttcttctc tggatatacc ttcaaacaca aaatggtcta tgaagacaca     2040
   cttaccctct tcccattctc aggagaaact gtcttcatgt caatggaaaa cccaggtctg     2100
   tgggttctgg ggtgccacaa ctcagacttt cggaacagag gcatgacagc cttactgaag     2160
   gtttctagtt gtaacaggaa cattgatgat tattatgagg acacatacga agatattcca     2220
   actcccctgc taaatgaaaa caatgtaatt aaacctagaa gcttctccca gaattcaagg     2280
   caccctagca ctaaggaaaa gcaattgaaa gccaccacaa ctccagaaaa tgacatagag     2340
   aagattgacc ttcaatctgg agaaagaaca cagctgatta aagcacaaag tgtctcctct     2400
   agtgatttgt tgatgctgtt gggacagaat cctactccac gtggactgtt cttatctgat     2460
   ctccgagagg ccacagatag agccgatgac cattcacgtg gagcaataga aagaaacaag     2520
   ggcccacctg aagtggcaag tctcagacca gagctccgtc acagtgagga cagagaattt     2580
   actcctgagc cagaactgca gttaagatta aatgagaatt tggggacaaa tacaacagta     2640
   gagttgaaga aacttgattt aaaaatttct agttcatcag acagtctaat gacttcacca     2700
   acaattccat cagataagtt ggcagcagct actgaaaaga caggttcctt aggaccccca     2760
   aatatgtcag ttcactttaa cagtcattta ggtaccattg tatttggcaa taattcatcc     2820
   caccttattc agtctggtgt acctttggaa ttgagtgaag aagataatga ttccaagttg     2880
   ttagaagcac ctttaatgaa tattcaagaa agttcactga gagaaaatgt attatcaatg     2940
   gagagtaata ggttatttaa agaagaaaga attcgtggac ctgcttcatt aatcaaagat     3000
   aatgctttat tcaaagttaa tatctcttcg gtaaagacaa acagggcacc agttaactta     3060
   acaactaata gaaagactcg tgttgctatc ccaacattat taattgagaa cagtacctca     3120
   gtctggcaag atattatgtt agaaaggaat actgagttta aagaagtaac ttctttgatt     3180
   cataatgaaa cgtttatgga cagaaatact acagctctgg ggctaaatca tgtgtcaaat 3240
   aaaactactt tatcaaaaaa tgtggaaatg gcccaccaaa aaaaagaaga ccctgtgcca 3300
   ctacgtgcag aaaatccaga tctatcatcc tccaagatac cgttcttgcc agattggata 3360
   aagacccatg gcaagaactc cctaagctct gagcaaaggc ccagtccaaa acaattaaca 3420
   tctttaggat cagaaaaatc tgtgaaagat cagaactttt tgtcagagga gaaggtggta 3480
   gtaggagagg atgaatttac gaaggacaca gaactccaag agatttttcc aaacaacaag 3540
   agcatatttt ttgctaactt ggctaatgtc caagaaaatg atacatacaa tcaagaaaaa 3600
   aaatctccgg aagagataga aagaaaggaa aaattaaccc aggagaatgt ggctttgcct 3660
   caggcacata ctatgattgg cactaagaac ttcctgaaga accttttctt actaagcact 3720
   aagcaaaatg tagcaggttt agaagagcag ccatatactc caatacttca agacaccagg 3780
   tcattaaatg attcgccaca tagtgaaggg attcatatgg ccaatttctc aaaaataagg 3840
   gaagaagcaa acttggaagg cttgggaaat caaacaaacc aaatggtaga gaggtttcca 3900
   agcactacga ggatgtcttc taatgcaagt cagcatgtta tcactcaacg tggtaagcgg 3960
   agtttgaaac aacccagact ctcacaagga gaaataaagt ttgaaaggaa ggttattgca 4020
   aatgacactt caacccagtg gtccaaaaac atgaactatt tggcccaggg aaccctcaca 4080
   cagatagagt ataatgagaa agaaaaaagg gccattactc agtcccccct atcagattgt 4140
   tctatgagga atcatgtcac cattcaaatg aatgactctg cattacccgt tgcaaaggaa 4200
   tcagcatctc catcagttag acatacagat ctgaccaaga tcccatccca acacaactct 4260
   tctcatcttc cagcatcagc ctgtaattat acctttagag agaggacttc tggagtccaa 4320
   gaaggcagtc atttcttaca agaagccaaa agaaataacc tctctttagc ctttgtaacc 4380
   ttaggaataa ctgaagggca aggaaagttc agctccctgg ggaaaagtgc cacaaaccaa 4440
   cccatgtaca agaaacttga aaacactgtt ctcttgcaac caggcttgtc cgaaacatct 4500
   gacaaagttg aattactttc tcaagttcat gttgatcaag aagactcttt ccctacaaaa 4560
   actagcaatg attctcctgg ccacctggat ctcatgggaa agatcttcct tcagaaaaca 4620
   cagggacctg ttaaaatgaa taaaacaaat agccctggaa aagtgccctt tctgaaatgg 4680
   gcaacagaaa gctctgaaaa gattccctcc aagctgctgg gtgtccttgc ttgggataac 4740
   cactatgata cccagatacc aagtgaagag tggaaatccc aaaaaaagtc acagacgaac 4800
   acagctttta aaaggaaaga caccattttg cccctgggcc cttgtgaaaa taatgattca 4860
   acagcagcaa taaatgaagg acaagataag ccccaaagag aagctatgtg ggcaaagcaa 4920
   ggagagcctg gaaggttgtg ctctcaaaac ccaccagtct caaaacacca tcaaagggaa 4980
   ataaccgtta ctactcttca gccagaggaa gacaaatttg agtatgatga caccttctca 5040
   attgaaatga agagagaaga ttttgacatc tacggcgact atgaaaatca gggcctccgc 5100
   agctttcaaa agaaaacacg acactatttc attgctgcag tggagcgtct ctgggattat 5160
   gggatgagta gatctcccca tatactaaga aacagggctc aaagtgggga tgtccagcag 5220
   ttcaagaagg tggttttcca ggaatttact gatggatcct ttactcagcc cttataccgt 5280
   ggagaactga acgaacactt gggactcttg gggccatata taagagcaga agttgaagac 5340
   aatatcgtgg taactttcaa aaaccaggcc tctcgtccct actccttcta ttctagtctt 5400
   atttcttatg acgaagatga gggacaagga gcagaaccta gaagaaagtt tgtcaaccct 5460
   aatgaaacca aaatttactt ttggaaagtg cagcatcata tggcacccac taaagatgag 5520
   tttgactgca aagcctgggc ttatttttct gatgttgatc tggagaaaga tgtgcactca 5580
   ggcttgattg gaccccttct gatctgccgc agtaacacac tgaaccctgc tcatgggaga 5640
   caagtgacag tgcaggagtt tgccctggtt ttcactatat tcgatgagac taagagctgg 5700
   tacttcactg aaaacctgga aaggaactgc agagctccct gcaatgtcca gaaggaggac 5760
   cctactctaa aagaaaactt ccgcttccat gcaatcaacg gctatgtgaa ggatacactc 5820
   cctggcttag taatggctca ggatcaaaag gttcgatggt atctgctcag catgggcagc 5880
   aacgaaaaca ttcattccat tcacttcagt ggacatgtgt tcactgtacg gaaaaaagag 5940
   gaatataaaa tggcagtcta caacctctat ccaggtgttt ttgagactgt ggaaatgcta 6000
   ccatcccaag ttggaatctg gcggatagaa tgccttatcg gcgagcacct gcaagccggg 6060
   atgagcactc tgtttctggt gtacagcaag aagtgtcaga ctccactggg gatggcttcc 6120
   ggacacatta gagattttca gattacagct tcaggacaat atggacagtg ggccccaaag 6180
   ctggccagac ttcattattc cggatcaatc aatgcctgga gcaccaagga tcccttttcc 6240
   tggatcaagg tggatctctt ggcaccgatg attattcacg gcatcatgac ccagggggcc 6300
   cgccagaagt tctccagcct ctacgtgtct cagtttatca tcatgtacag tctggatggc 6360
   aacaagtggc acagttaccg agggaattcc acggggacct taatggtctt ctttggcaac 6420
   gtggattcat ctgggatcaa acacaatatt tttaaccctc cgattattgc tcagtacatc 6480
   cgtttgcacc caacccatta cagcatccgc agcactcttc gcatggagct cttgggctgt 6540
   gacttcaaca gttgcagcat gccgctgggg atggagagta aagcaatatc agatgctcag 6600
   atcactgcct cgtcctacct aagcagtatg cttgccactt ggtctccttc ccaagcccgg 6660
   ctgcacctgc agggcaggac taatgcctgg agacctcagg caaataaccc aaaagagtgg 6720
   ctgcaagtgg acttccggaa gaccatgaaa gtcacaggaa taaccaccca gggggtgaaa 6780
   tctctcctca tcagcatgta tgtgaaggag ttcctcatct ccagtagtca agatggccat 6840
   aactggactc tgtttcttca gaatggcaaa gtcaaggtct tccagggaaa ccgggactcc 6900
   tccacgcctg tgcggaaccg tctcgaaccc ccgctggtgg ctcgctacgt gcgcctgcac 6960
   ccgcagagct gggcgcacca catcgccctg aggctggagg tcctgggctg cgacacccag 7020
   cagcccgcct ga 7032
<210> 2
   <211> 171
   <212> DNA
   <213> Canis lupus familiaris
<220>
   <221> source
   <222> 1..171
   <223> /mol_type="DNA" /organism="Canis lupus familiaris"
<400> 2
   gggatccctg ggtggcgcag cggtttggcg cctgcctttg gcccagggcg cgatcctgga 60
   gacccgggat cgaatcccac atcgggctcc tggtgcatgg agcctgcttc tctctctgcc 120
   tgtgtctctg cctctctctc tctctctgtc tgtgacttaa ataaataaat a 171
<210> 3
   <211> 218
   <212> DNA
   <213> Canis lupus familiaris
<220>
   <221> source
   <222> 1..218
   <223> /mol_type="DNA" /organism="Canis lupus familiaris"
<400> 3
   gggatccctg ggtggcgcag cggtttggcg cctgcctttg gcccagggcg cgatcctgga 60
   gacccgggat cgaatcccac atcgggctcc tggtgcatgg agcctgcttc tctctctgcc 120
   tgtgtctctg cctctctctc tctctctgtc tgtgacttaa ataaataaat aataaataaa 180
   atatttaaaa aaaaaaaaaa aaaaaaaaaa aaaaaaaa 218
<210> 4
   <211> 234
   <212> DNA
   <213> Canis lupus familiaris
<220>
   <221> source
   <222> 1..234
   <223> /mol_type="DNA" /organism="Canis lupus familiaris"
<400> 4
   gggatccctg ggtggcgcag cggtttggcg cctgcctttg gcccagggcg cgatcctgga     60
   gacccgggat cgaatcccac atcgggctcc tggtgcatgg agcctgcttc tctctctgcc    120
   tgtgtctctg cctctctctc tctctctgtc tgtgacttaa ataaataaat aataaataaa    180
   atatttaaaa aaaaaaaaaa aaaaaaaaaa aaaaaaaata aaaatttcta gttc          234
<210> 5
   <211> 417
   <212> DNA
   <213> Canis lupus familiaris
<220>
   <221> source
   <222> 1..417
   <223> /mol_type="DNA" /organism="Canis lupus familiaris"
<220>
   <221> misc_feature
   <222> 317-332
   <223> /note="duplication site"
<220>
   <221> misc_feature
   <222> 83..98
   <223> /note="duplication site"
<220>
   <221> misc_feature
   <222> 99..316
   <223> /note="SINE insertion with poly-A"
<400> 5
   tttactcctg agccagaact gcagttaaga ttaaatgaga atttggggac aaatacaaca     60
   gtagagttga agaaacttga tttaaaaatt tctagttcgg gatccctggg tggcgcagcg    120
   gtttggcgcc tgcctttggc ccagggcgcg atcctggaga cccgggatcg aatcccacat    180
   cgggctcctg gtgcatggag cctgcttctc tctctgcctg tgtctctgcc tctctctctc    240
   tctctgtctg tgacttaaat aaataaataa taaataaaat atttaaaaaa aaaaaaaaaa    300
   aaaaaaaaaa aaaaaataaa aatttctagt tcatcagaca gtctaatgac ttcaccaaca    360
   attccatcag ataagttggc agcagctact gaaaagacag gttccttagg accccca       417
<210> 6
   <211> 25
   <212> DNA
   <213> artificial sequences
<220>
   <221> source
   <222> 1..25
   <223> /mol_type="DNA" /note="Forward primer FVIII" /organism="artificial sequences"
<400> 6
   caacatgatg gtatggaagc ttatg 25
<210> 7
   <211> 25
   <212> DNA
   <213> artificial sequences
<220>
   <221> source
   <222> 1..25
   <223> /mol_type="DNA" /note="FVIII reverse primer" /organism="artificial sequences"
<400> 7
   caaagctaac tacgtccatg tcaga 25
<210> 8
   <211> 25
   <212> DNA
   <213> artificial sequences
<220>
   <221> source
   <222> 1..25
   <223> /mol_type="DNA" /note="SINE forward primer" /organism="artificial sequences"
<400> 8
   gagaatttac tcctgagcca gaact 25
<210> 9
   <211> 22
   <212> DNA
   <213> artificial sequences
<220>
   <221> source
   <222> 1..22
   <223> /mol_type="DNA" /note="SINE reverse primer" /organism="artificial sequences"
<400> 9
   ccagggatcc cgaactagaa at 22
<210> 10
   <211> 20
   <212> DNA
   <213> artificial sequences
<220>
   <221> source
   <222> 1..20
   <223> /mol_type="DNA" /note="beta-actin forward primer" /organism="artificial sequences"
<400> 10
   tcgctgacag gatgcagaag 20
<210> 11
   <211> 20
   <212> DNA
   <213> artificial sequences
<220>
   <221> source
   <222> 1..20
   <223> /mol_type="DNA" /note="beta-actin reverse primer" /organism="artificial sequences"
<400> 11
   gtggacagtg aggccaggat 20
<210> 12
   <211> 18
   <212> DNA
   <213> artificial sequences
<220>
   <221> source
   <222> 1..18
   <223> /mol_type="DNA" /note="GAPDH forward primer" /organism="artificial sequences"
<400> 12
   tccacccacg gcaaattc 18
<210> 13
   <211> 21
   <212> DNA
   <213> artificial sequences
<220>
   <221> source
   <222> 1..21
   <223> /mol_type="DNA" /note="GAPDH reverse primer" /organism="artificial sequences"
<400> 13
   gcatcacccc atttgatgtt g 21